# FASCICULE DE BREVET EUROPEEN

(11) **EP 3 282 980 B1**
(45) Date de publication et mention de la délivrance du brevet: **17.08.2022**
(21) Numéro de dépôt: 16718436.5
(22) Date de dépôt: 07.04.2016
(51) Int. Cl.: A61B 17/70

(54) **SYSTEME ET PIECE D'ANCRAGE VERTEBRAL**
WIRBELFIXIERUNGSVORRICHTUNG
VERTEBRAL FIXATION DEVICE

(30) Priorité: 17.04.2015 FR 1553424
(43) Date de publication de la demande: 21.02.2018
(73) Titulaire: Implanet, 33650 Martillac (FR)
(72) Inventeur: LE COUËDIC, Régis, 33000 Bordeaux (FR); PASQUET, Denis, 13100 Aix En Provence (FR)
(74) Mandataire: Benech, Frédéric
(86) Numéro de dépôt international: PCT/FR2016/050799
(87) Numéro de publication internationale: WO 2016/166448

(56) Documents cités:
- EP-A1- 0 928 603
- US-A1- 2008 208 256
- US-A1- 2012 221 056
- US-A1- 2012 259 369
- US-A1- 2013 072 983

## Description

La présente invention concerne un système d'ancrage vertébral comprenant deux pièces d'ancrage comportant chacune un élément pédiculaire propre à être engagé dans un pédicule de vertèbre et reliées l'une à l'autre par un élément de liaison.

Elle concerne également les pièces d'ancrage proprement dites et décrit un procédé d'ancrage vertébral pour solidariser deux vertèbres adjacentes entre elles.

Elle trouve une application particulièrement importante bien que non exclusive dans le domaine de la consolidation de la colonne vertébrale en cas de lésion, et plus particulièrement lorsque l'une des vertèbres est de mauvaise qualité, par exemple due à l'ostéoporose ou à un accident.

On sait que les disques intervertébraux peuvent être l'objet de tassement, d'hernie discale ou de dégénérescence arthrosique intervertébrale.

Pour soulager les patients, il existe alors des techniques nécessitant une intervention chirurgicale.

Une première technique consiste à remplacer le disque endommagé par une prothèse de disque intervertébral. Un tel remplacement est délicat à effectuer par le chirurgien et présente un risque de désolidarisation sous l'effet des forces de cisaillements importantes qui s'exercent notamment lorsque la prothèse est en position de flexion maximale.

Une deuxième technique consiste à réaliser une arthrodèse intervertébrale, opération permettant de fusionner les deux vertèbres adjacentes au disque endommagé. Il y a alors blocage de la dégradation du fait de la suppression de toute mobilité entre les deux vertèbres concernées.

C'est une amélioration de cette technique qui est l'objet de la présente l'invention.

On sait qu'une telle technique implique l'utilisation d'un dispositif de stabilisation des deux vertèbres comportant des vis destinées à être vissées dans les vertèbres et solidarisées entre elles par un organe rigide de liaison. Il est ainsi possible d'éviter que des contraintes mécaniques trop importantes ne soient appliquées au disque intervertébral.

Mais cette technique présente des inconvénients nécessitant en particulier de visser dans les vertèbres ce qui est une opération parfois impossible si ces dernières sont en mauvais état et/ou insuffisamment larges au niveau de la fixation.

Le document US 2013/072983 décrit un dispositif d'ancrage avec une pièce d'ancrage.

On connaît (FR 2 913 330) des dispositifs d'ancrage réalisés par l'assemblage de deux pièces d'ancrage comportant chacune un clou pédiculaire et reliées entre elles par une liaison rigide.

Un tel système qui assure une bonne stabilité dans l'os dégradé présente cependant l'inconvénient d'un montage compliqué, les clous pédiculaires pouvant de plus présenter des inclinaisons incompatibles du fait de la configuration de la vertèbre.

On connait également un dispositif d'ancrage comprenant deux pièces d'ancrage reliées par une bande passant à travers des œillets (US 2008/208256) ou par une barre (EP 0 928 603).

La présente invention vise à pallier ces inconvénients et à fournir un système, une pièce et un procédé d'ancrage vertébral répondant mieux que ceux antérieurement connus aux exigences de la pratique, notamment en ce qu'elle permet une plus grande flexibilité, en ce qu'elle évite tout élément intrusif dans un os de vertèbre fragilisé pour peu qu'une vertèbre solide existe à côté de la vertèbre abimée, et en ce qu'elle présente une bonne solidité et ce pour un coût faible et/ou moindre qu'avec les dispositifs de l'art antérieur.

Elle présente également l'avantage d'un maniement aisé permettant sa mise en place facile par le chirurgien.

Dans ce but l'invention propose notamment un système d'ancrage vertébral comprenant deux pièces d'ancrage comportant chacune un élément pédiculaire propre à être engagé dans un pédicule de vertèbre et reliées l'une à l'autre par un élément de liaison, caractérisé en ce que l'élément de liaison est une bande souple, en ce que les éléments pédiculaires sont des vis, en ce que chaque pièce d'ancrage comporte une tête de fixation sur une tige solidaire de moyens de blocage réglable de la bande souple en tension par rapport à la tête et en ce qu'il comporte deux tiges correspondantes auxdites têtes de fixation.

L'idée du système est de contrôler les mouvements de la vertèbre dégradée par rapport à la vertèbre adjacente plus solide, par une liaison sur l'épineuse de la vertèbre du dessus ou du dessous (sur son apophyse) grâce à une mise en tension des deux côtés de la bande.

Ici le coincement de la tresse ou bande par les moyens de blocage va pouvoir autoriser le trajet longitudinal de celle-ci en reproduisant ce que font naturellement les ligaments de la colonne.

L'amplitude des mouvements va ainsi pouvoir être limitée sans être complètement bloquée, ce qui évite en partie de reporter tous les efforts sur le disque libre adjacent aux vertèbres bloquées en mouvement entre elles, qui autrement engendre le phénomène bien connu d'effet « charnière », risquant cette fois-ci d'endommager le disque adjacent encore en bon état.

Avec l'invention on va de plus créer une force de rappel sur les vis, force qui les ramène dans leur logement et évite ainsi les inconvénients des ciments consolidant en général utilisés avec les vis de l'art antérieur. De tels ciments posent des problèmes de température (nécessaire à une bonne polymérisation du ciment) et entrainent par ailleurs une impossibilité de ré intervention sur la vis une fois bloquée dans la masse.

Dans des modes de réalisation avantageux, on a par ailleurs et/ou de plus recours à l'une et/ou à l'autre des dispositions suivantes :
- la tête de fixation de la pièce d'ancrage est solidaire d'une bague qui est munie des moyens de blocage ;
- la tête de fixation comprend un corps en forme de coupelle traversée latéralement par la tige perpendiculairement à la vis pédiculaire et munie d'un taraudage interne dans sa partie supérieure, de fixation de la tige par le biais d'une vis de compression, et la bague comprend un premier orifice traversant clipsable par déformation sur la périphérie supérieure de la tête de fixation, un deuxième orifice latéral traversant de passage de la bande et un troisième orifice d'insertion des moyens de blocage de la bande dans la bague ;
- la tête de fixation comprend une partie supérieure cylindrique ou en portion cylindrique d'un premier diamètre, le premier orifice étant cylindrique de diamètre complémentaire au premier diamètre avec lequel il est agencé pour coopérer à frottement avant vissage de la vis de compression et avec lequel il est agencé pour se bloquer lors du serrage de ladite vis.

Ce blocage provient de l'élargissement ou éloignement des parois du taraudage interne de la partie supérieure de la tête du fait de la compression exercée par la tête de la vis de compression elle-même sur ses bords.

Il en résulte par répercussion une compression de la paroi externe de tête sur la paroi interne du premier orifice et un blocage en conséquence en compression latérale ;
- la tête comprend une partie supérieure munie d'une gorge ou d'une nervure de rétention et le premier orifice traversant de la bague est muni d'une nervure ou d'une gorge de forme correspondante agencée pour être encastrée en force l'une dans l'autre;
- les moyens de blocage comprennent une pièce de maintien insérable dans le troisième orifice de la bague, la pièce de maintien étant au moins en partie en forme de coin écrasable, de blocage de la bande passant par le deuxième orifice ;
- la pièce de maintien présente une section transversale en forme de diapason dont les branches comprennent des dents extérieures anti-retour et dont le manche présente une extrémité arrondie, et le troisième orifice présente des parois latérales munies de dents anti-retour complémentaires de celle de la pièce de maintien et un fond de forme complémentaire à celle de l'extrémité du manche, de coincement de la bande entre les deux ;
- la vis pédiculaire présente une extrémité ronde ou oblongue montée pivotante en rotation dans la partie inférieure de la tête et la tête comporte une structure de jonction entre la paroi cylindrique de la tige et ladite extrémité de vis.

L'invention concerne aussi une pièce d'ancrage telle que décrite ci-dessus. Un procédé d'ancrage mettant en œuvre un système tel que décrit ci-dessus est également détaillé.

Elle est utilisée dans un procédé d'ancrage vertébral pour solidariser deux vertèbres adjacentes entre elles dans lequel on visse deux vis pédiculaires dans les pédicules en vis à vis d'une vertèbre, on glisse une tige au travers d'une tête de fixation de chaque vis sur ladite tige, et on visse une vis de pression sur la tige au travers de la partie supérieure de la tête, caractérisé en ce que avant de visser la vis de pression, on clipse sur la périphérie supérieure de chaque tête une bague comprenant un premier orifice traversant clipsable par déformation sur la dite périphérie supérieure et comprenant un deuxième orifice latéral traversant de passage d'une bande et un troisième orifice d'insertion de moyens de blocage de la bande sur ladite tête de fixation, on place la bande souple sur la vertèbre adjacente et dans les moyens de blocage de chaque tête de fixation, on bloque ladite bande sur une tête et on commence à mettre ladite bande souple en tension en tirant de l'autre côté, puis on met le tout en tension avant de bloquer définitivement la bande par rapport à la tête restante.

On sécurise ainsi complètement mais avec douceur le mouvement de la vertèbre adjacente par rapport à celui de la vertèbre de base, en empêchant la flexion de l'une par rapport à l'autre, et ce dans le sens de fonctionnement de la colonne vertébrale.

L'invention sera mieux comprise à la lecture de la description qui suit de modes de réalisation donnés ci-après à titre d'exemples non limitatifs. La description se réfère aux dessins qui l'accompagnent dans lesquels :
La figure 1 est une vue en perspective axionométrique d'une pièce d'ancrage selon un premier mode de réalisation de l'invention.

La figure 1A est une vue en coupe selon I_{A} - I_{A} de la figure 1.

La figure 1B est une vue en coupe selon I_{B} - I_{B} de la figure 1.

La figure 1C est une vue en coupe partielle du type de la coupe selon I_{B} - I_{B}, d'une variante de la pièce de la figure 1.

La figure 2 est une vue en perspective de la bague de la pièce de la figure 1.

La figure 3 illustre schématiquement en vue de dessus le système d'ancrage utilisant deux pièces d'ancrage selon la figure 1, deux tiges et la bande souple en tension autour de l'apophyse d'une vertèbre.

La figure 4 est une vue en perspective d'un système comprenant deux pièces d'ancrage selon le mode de réalisation de l'invention plus particulièrement décrit ici, en position sur deux vertèbres adjacentes. A noter qu'on a ici fait figurer qu'une vis pédiculaire de fixation par tige, chaque tige devant bien entendu en comporter plusieurs pour bloquer les mouvements d'au moins deux vertèbres adjacentes.

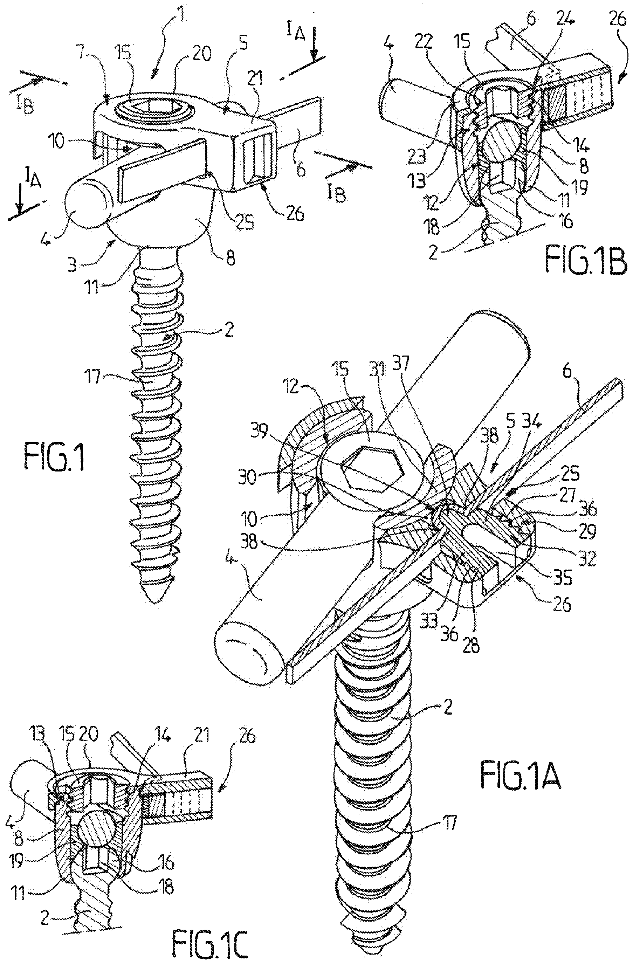

La figure 1 montre une pièce d'ancrage 1 comportant une vis pédiculaire 2 propre à être vissée dans un pédicule de vertèbre (non représenté).

La pièce comporte une tête de fixation 3 sur une tige 4 solidaire de moyens 5 de blocage réglable d'une bande souple 6 en tension.

La bande 6 est plate par exemple en polyester tressé de 1 à 3 mm d'épaisseur et de 6 mm de large.

Les moyens 5 de blocage comprennent une bague 7 solidaire de la tête 3, qui comprend un corps 8 en forme de coupelle évidée percée en sa partie inférieure ou fond 9 pour laisser passer la vis pédiculaire 2, et comporte deux encoches latérales 10, ouvertes vers le haut, de passage latéral de la tige 4 qui vient s'y encastrer.

Le corps 8 par exemple en titane, est cylindrique et comprend un fond arrondi et un évidement intérieur cylindrique 12 de passage et de blocage dans le sens axial de la vis.

Sa partie supérieure 13 comprend un taraudage interne 14 de fixation de la tige, introduite dans les encoches latérales 10, par une vis 15 de compression qui vient donc comprimer la tige sur la tête 16 de la vis pédiculaire 2.

Celle-ci comprend un corps 17 de vissage muni d'un pas de vis connu en lui-même.

La tête 16 de la vis (cf. figure 1B) est munie d'une encoche 18 de vissage par un outil. Elle est partiellement sphérique et vient s'appuyer sur le fond 11 de la coupelle du corps 8 de la tête de fixation 3 avec qui elle coopère à frottement par rotation et en butée longitudinale.

Après vissage de la vis 2 préalablement introduite dans la pièce d'ancrage 1, et après introduction de la tige 4, on met en place la bague 7, et ce après introduction au-dessus de la tête de vis d'une pièce 19 de jonction/guidage/soutien en forme de demi-cylindre d'un côté (tige) et d'anneau de l'autre (tête de vis).

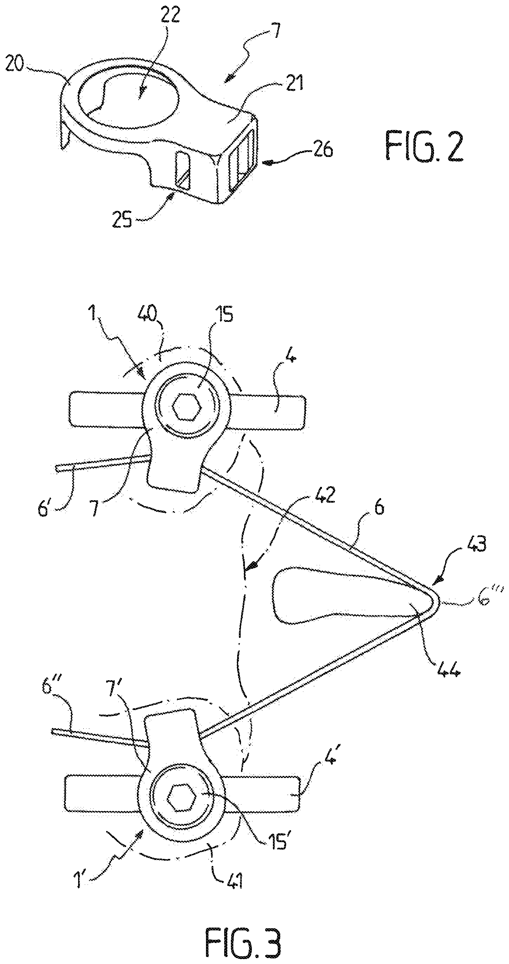

La bague 7 (cf. également figure 2) est une pièce par exemple en titane, de section transversale en forme de trou de serrure, avec une première portion 20 cylindrique et une seconde portion 21 parallélépipédique de plus petite largeur que le diamètre, ou sensiblement parallélépipédique.

La première portion 20 cylindrique (ou sensiblement cylindrique) est munie d'un alésage cylindrique interne 22 ou premier orifice comprenant une partie supérieure 23 rétrécie formant cornière de blocage en déplacement axial avec le dessus 24 de la tête de fixation.

L'alésage interne 22 coopère à frottement en contact cylindre/cylindre avec la surface externe de la partie supérieure cylindrique 13 du corps 8.

Une fois la bague insérée sur cette partie supérieure en frottement, la vis 15 de serrage en compression est vissée.

De par ce vissage, la tête de la vis 15 étant conçue pour, on réalise une légère expansion du volume qu'elle occupe lors du serrage, par exemple de l'ordre de 0,2 mm, qui déforme en compression latérale l'alésage de l'attache, ce qui bloque tout mouvement de la bague par rapport à l'attache.

La figure 1C montre un autre mode de réalisation où le premier orifice 22 de la bague comprend une nervure encliquetée en force dans une rainure ou gorge correspondante.

La bague 7 comprend un deuxième orifice 25, latéral, traversant, de passage de la bande 6 (en forme de fente verticale de largeur et épaisseur un peu plus grandes (par exemple avec un jeu de 1 mm) que celles de la bande 6 pour lui permettre son passage facile.

La bague 7 comprend de plus un troisième orifice 26 traversant la portion 21 perpendiculairement par rapport au deuxième orifice 25. La portion 21 comprend une première partie 27 (cf. figure 1A) parallélépipédique comprenant des dents anti retour 28 sur deux de ses faces latérales internes 29 en vis à vis (par exemple trois dents), par exemple sur les faces latérales situées dans des plans parallèles à l'axe de la vis 2 et, de l'autre côté de l'orifice traversant 25 par rapport à cette première partie 27, une deuxième partie 30, parallélépipédique de plus petite largeur, mais de même hauteur que la première partie 27 d'orifice, par exemple débouchant sur la périphérie de la face externe 31 de la partie supérieure de la pièce de fixation 3.

Les moyens 5 de blocage de la bande 6 comprennent par ailleurs une pièce de maintien 32 en forme de diapason dont les branches sont écrasables et/ou insérables en se déformant dans le troisième orifice 26.

Plus précisément la pièce 32 comprend une section transversale (cf. figure 1A) en forme de diapason comportant deux branches 33 et 34 séparées par un évidement 35, munies elles-mêmes, sur leurs faces extérieures en vis à vis des faces latérales internes 29 de dents anti retour 36 complémentaires des dents 28 avec lesquelles elles vont s'encastrer par encliquetage progressif lorsque la pièce 32 est insérée en force dans la première partie 27.

La pièce 32 comprend par ailleurs une langue en excroissance formant un manche 37 d'extrémité du diapason qui vient s'insérer dans la deuxième partie 30 de l'orifice pour venir coincer la bande 6 entre elle et les bords latéraux 38 et le fond 39 de ladite deuxième partie lorsque la pièce 32 est complètement encastrée dans le troisième orifice 26.

Pour ce faire un léger jeu va être laissé entre les parois du manche et celle de la deuxième partie d'orifice, pour permettre un écrasement suffisant de la bande et son blocage en position avec un dimensionnement calculé à la portée de l'homme du métier.

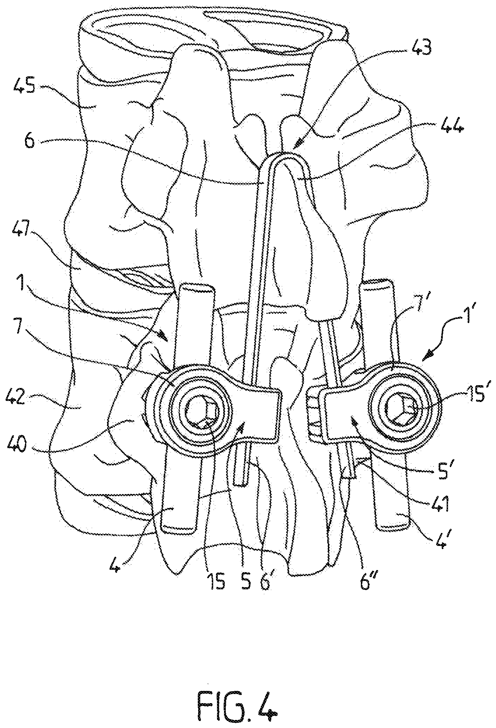

Les figures 3 et 4 illustrent le système d'ancrage selon le mode de réalisation de l'invention plus particulièrement décrit ici, utilisant deux pièces d'ancrage 1, 1' dans les deux pédicules opposés 40 et 41 de la vertèbre 42, chaque pièce étant munie de vis 2, 2' et fixée sur les tiges 4, 4' par le biais des vis de compression 15, 15'.

La bande 6 est bloquée de part et d'autre par les moyens 5, 5' de blocage et vient s'encastrer en 43 sur l'épiphyse 44 de la vertèbre 45 (cf. figure 4).

On va maintenant décrire la mise en œuvre de l'ancrage avec le système selon le mode de réalisation de l'invention plus particulièrement décrit ici en faisant référence aux figures 1A et 4.

Le chirurgien qui veut soulager le disque intervertébral 47 entre la vertèbre 42 (solide) et la vertèbre 45 (fragile) commence par fixer les vis pédiculaires préalablement munies de leur tête de fixation dans les pédicules 40 et 41 de la vertèbre 42, et ce de façon connue en elle-même, en vissant lesdites vis au travers des têtes, par le biais d'un tourne vis ou outil qui attaque l'encoche 18.

Il vient ensuite encastrer les tiges 4, 4' sur les têtes, puis enclipser les bagues 7, 7' sur la périphérie supérieure de chaque tête.

Préalablement ou ensuite, alors que les bagues conservent encore un certain degré de liberté en rotation par rapport à la tête de fixation correspondante, il glisse dans le troisième orifice, les portions d'extrémité respectivement 6' et 6" de la bande 6.

Il vient alors placer la partie centrale 6‴ de la bande sur l'arête pinéale 44 de la vertèbre 45 adjacente.

Puis, d'une part il bloque la tête 3 sur la tige 4 en vissant la vis de compression 15 et d'autre part il bloque la portion d'extrémité 6' de la bande avec les moyens 8 de blocage, en insérant en totalité la pièce de maintien 32 dans le troisième orifice de la bague correspondante, les dents 28 de l'orifice coopérant en s'encastrant progressivement et par déformation puis reprise de leur emplacement dans les dents 36 du diapason, du fait de l'élasticité relative des branches dudit diapason.

Les dimensions des éléments sont quant à elles conçues pour coincer la bande dans le recoin formé par la deuxième partie 30 du troisième orifice de façon inamovible et calculées de façon à la portée de l'homme du métier.

Le chirurgien tend alors avec un outil de type connu la bande 6 en tirant sur la portion d'extrémité 6" au travers des moyens de blocage 8' non encore contraint, la tête 3' de fixation ayant quant à elle été bloquée sur la tige 41 par vissage de la vis de compression 15'.

Puis une fois la tension sur l'arête 44 requise obtenue (par exemple par le biais d'un tenseur dynamométrique) l'extrémité 6" est définitivement bloquée par rapport à la tête 3'.

Comme il va de soi et comme il résulte également de ce qui précède, la présente invention n'est pas limitée aux modes de réalisation plus particulièrement décrits. Elle en embrasse au contraire toutes les variantes et notamment celles où les moyens de blocage sont différents, celles ou les vis pédiculaires sont à double filets ou encore celles ou l'encliquetage des bagues sur la tête de fixation se fait par contact cône/cône, par exemple avec un angle de conicité du type cône morse et avec une languette de blocage déformable en partie inférieure.

## Revendications

1. Système d'ancrage vertébral comprenant deux pièces d'ancrage (1) comportant chacune un élément pédiculaire (2) propre à être engagé dans un pédicule de vertèbre et reliées l'une à l'autre par un élément de liaison, qui est une bande (6) souple, les éléments pédiculaires (2) étant des vis, chaque pièce d'ancrage (1) comport ant une tête de fixation (3) sur une tige (4), et la tête de fixation (3) comprenant un corps (8) en forme de coupelle traversée latéralement par la tige (4) perpendiculairement à la vis pédiculaire (2) et muni d'un taraudage interne (14) dans sa partie supérieure (13), de fixation de la tige par le biais d'une vis (15) de compression, **caractérisé en ce que** la tête (3) est solidaire d'une bague (7) et **en ce que** la bague (7) est munie de moyens (5) de blocage réglable de la bande souple en tension par rapport à la tête (3) et comprend un premier orifice (22) traversant clipsable par déformation sur la périphérie supérieure (13) de la tête de fixation, un deuxième orifice (25) latéral traversant de passage de la bande (6) et un troisième orifice (26) d'insertion des moyens de blocage de la bande (6) dans la bague (7) et **en ce qu'**il comporte deux tiges (4, 4') correspondantes auxdites têtes de fixation.

2. Système selon la revendication 1, **caractérisé en ce que** la tête de fixation (3) comprend une partie supérieure cylindrique ou en portion cylindrique d'un premier diamètre, le premier orifice (22) est cylindrique de diamètre complémentaire au premier diamètre avec lequel il est agencé pour coopérer à frottement avant vissage de la vis (15) de compression et avec lequel il est agencé pour se bloquer lors du serrage.

3. Système selon la revendication 1, **caractérisé en ce que** la tête de fixation (3) comprend une partie supérieure munie d'une gorge ou d'une nervure de rétention et **en ce que** le premier orifice (22) traversant de la bague est muni d'une nervure ou d'une gorge de forme correspondante agencée pour être encastrée en force l'une dans l'autre.

4. Système selon l'une quelconque des revendications 1 à 3, **caractérisé en ce que** les moyens (5) de blocage comprennent une pièce de maintien (32) insérable dans le troisième orifice (26) de la bague, la pièce de maintien étant au moins en partie en forme de coin écrasable, de blocage de la bande (6) passant par le deuxième orifice (25).

5. Système selon la revendication 4, **caractérisé en ce que** la pièce de maintien (32) présente une section transversale en forme de diapason dont les branches (33, 34) comprennent des dents (36) extérieures anti-retour et dont le manche (37) présente une extrémité arrondie, et **en ce que** le troisième orifice (26) présente des parois latérales munies de dents (28) anti-retour complémentaires de celles de la pièce de maintien (32) et un fond de forme complémentaire à celle de l'extrémité du manche, de coincement de la bande (6) entre les deux.

6. Système selon l'une quelconque des revendications précédentes, **caractérisé en ce que** la vis pédiculaire (2) présente une extrémité (16) ronde ou oblongue montée pivotante en rotation dans la partie inférieure de la tête de fixation et **en ce que** la tête de fixation comporte une structure (19) de jonction entre la paroi cylindrique de la tige et ladite extrémité de vis.

## Patentansprüche

1. System zur Wirbelverankerung mit zwei Verankerungsstücken (1) mit jeweils einem Pedikelelement (2), das geeignet ist, in einen Wirbelpedikel eingeführt zu werden, und die miteinander über ein Verbindungselement verbunden sind, welches ein flexibles Band (6) ist, wobei die Pedikelelemente (2) Schrauben sind, wobei jedes Verankerungsstück (1) einen Kopf (3) zur Befestigung an einem Stift (4) aufweist und der Befestigungskopf (3) einen Körper (8) umfasst, welcher die Form einer Schale aufweist, die seitlich von dem Stift (4) senkrecht zu der Pedikelschraube (2) durchquert wird und welcher in seinem oberen Bereich (13) mit einem Innengewinde (14) versehen ist, für die Befestigung des Stifts durch eine Kompressionsschraube (15), **dadurch gekennzeichnet, dass** der Kopf (3) mit einem Ring (7) fest verbunden ist und dass der Ring (7) mit Mitteln (5) zur verstellbaren Arretierung des gespannten flexiblen Bands in Bezug auf den Kopf (3) versehen ist und eine erste Durchgangsöffnung (22) umfasst, die durch Verformung auf den oberen Umfang (13) des Befestigungskopfes aufclipsbar ist, eine zweite seitliche Durchgangsöffnung (25) für die Durchführung des Bands (6) und eine dritte Durchgangsöffnung (26) zum Einführen der Arretierungsmittel des Bands (6) in den Ring (7), und dass es zwei mit den Befestigungsköpfen korrespondierende Stifte (4, 4') aufweist.

2. System nach Anspruch 1, **dadurch gekennzeichnet, dass** der Befestigungskopf (3) einen zylindrischen oder zylinderabschnittsförmigen oberen Bereich mit einem ersten Durchmesser aufweist, die erste Durchgangsöffnung (22) zylindrisch mit einem Durchmesser ist, der komplementär zum ersten Durchmesser ist, mit dem er angeordnet ist, um vor Eindrehen der Kompressionsschraube (15) reibschlüssig zusammenzuwirken und mit dem er angeordnet ist, um sich beim Anziehen zu blockieren.

3. System nach Anspruch 1, **dadurch gekennzeichnet, dass** der Befestigungskopf (3) einen oberen Bereich mit einer Rückhaltenut bzw. -rippe umfasst und die erste Durchgangsöffnung (22) des Rings mit einer Nut bzw. Rippe mit korrespondierender Form versehen ist, welche angeordnet ist, um mit Kraftschluss ineinander gesteckt zu werden.

4. System nach einem beliebigen der Ansprüche 1 bis 3, **dadurch gekennzeichnet, dass** die Arretierungsmittel (5) ein in die dritte Öffnung (26) des Rings einsetzbares Haltestück (32) umfassen, wobei das Haltestück mindestens teilweise als zusammendrückbarer Keil zur Arretierung des durch die zweite Öffnung (25) durchgeführten Bands (6) ausgeformt ist.

5. System nach Anspruch 4, **dadurch gekennzeichnet, dass** das Haltestück (32) einen stimmgabelförmigen Querschnitt aufweist, dessen Zacken (33, 34) Rückschlag-Außenzähne (36) aufweisen und dessen Griff (37) ein abgerundetes Ende aufweist, und dass die dritte Öffnung (26) Seitenwände mit Rückschlagzähnen (28), die komplementär zu denjenigen des Haltestücks (32) sind, und einen Boden aufweist, der komplementär zur Form des Endes des Griffs ausgeformt ist, um das Band (6) zwischen beiden zu klemmen.

6. System nach einem beliebigen der vorhergehenden Ansprüche, **dadurch gekennzeichnet, dass** die Pedikelschraube (2) ein rundes oder längliches Ende (16) aufweist, das drehkippbar im unteren Bereich des Befestigungskopfes montiert ist, und dass der Befestigungskopf eine Verbindungsstruktur (19) zwischen der zylindrischen Wand des Stifts und dem Schraubenende aufweist.

## Claims

1. Vertebral anchoring system comprising two anchoring components (1) which each have a pedicle element (2) for engagement in a vertebral pedicle and are connected to each other by a linking element which is a flexible band (6), the pedicle elements (2) being screws, each anchoring component (1) having a head (3) for fixing to a rod (4), and the fixing head (3) comprising a body (8) which is in the shape of a cup, traversed laterally by the rod (4) perpendicularly with respect to the pedicle screw (2), and is provided with an internal thread (14) in its upper part (13), for fixing the rod by way of a compression screw (15), **characterized in that** the head (3) is rigidly connected to a ring (7) and **in that** the ring (7) is provided with means (5) for adjustably blocking the flexible band under tension with respect to the head (3) and comprises a first through-orifice (22) which can be clipped by deformation onto the upper periphery (13) of the fixing head, a second lateral through-orifice (25) for passage of the band (6), and a third orifice (26) for insertion of the means for blocking the band (6) in the ring (7), and **in that** it has two rods (4, 4') corresponding to said fixing heads.

2. System according to Claim 1, **characterized in that** the fixing head (3) comprises an upper part which is cylindrical or shaped as a portion of a cylinder with a first diameter, the first orifice (22) is cylindrical with a diameter matching the first diameter, with which it is designed to cooperate by friction before the compression screw (15) is screwed in, and with which it is designed to be blocked during the clamping.

3. System according to Claim 1, **characterized in that** the fixing head (3) comprises an upper part provided with a retaining groove or rib, and **in that** the first through-orifice (22) of the ring is provided with a rib or groove of corresponding shape designed to be engaged with force one inside the other.

4. System according to any one of Claims 1 to 3, **characterized in that** the blocking means (5) comprise a holding component (32) that is insertable into the third orifice (26) of the ring, the holding component being at least partially in the shape of a squeezable wedge, for blocking the band (6) passing through the second orifice (25) .

5. System according to Claim 4, **characterized in that** the holding component (32) has a cross section in the shape of a tuning fork, of which the prongs (33, 34) comprise external non-return teeth (36), and of which the handle (37) has a rounded end, and **in that** the third orifice (26) has side walls, which are provided with non-return teeth (28) matching those of the holding component (32), and a bottom with a shape matching that of the end of the handle, for wedging the band (6) between the two.

6. System according to any one of the preceding claims, **characterized in that** the pedicle screw (2) has a round or oblong end (16) mounted pivotably in rotation in the lower part of the fixing head, and **in that** the fixing head has a joining structure (19) between the cylindrical wall of the rod and said screw end.
